# EUROPEAN PATENT APPLICATION

(11) **EP 4 541 178 A1**
(43) Date of publication of application: **23.04.2025**
(21) Application number: 22946985.3
(22) Date of filing: 08.11.2022
(51) Int. Cl.: A01G 7/06, A01N 43/10, A01G 22/15, A01G 22/05, A01G 31/00, A23L 33/105, A23L 29/00

(54) **METHOD FOR INCREASING FLAVONOID CONTENT IN PLANT THROUGH TREATMENT WITH LOW-MOLECULAR-WEIGHT SULFUR COMPOUND**

(30) Priority: 15.06.2022 KR 20220072847
(71) Applicant: Republic of Korea Management: Rural Development Administration, Jeollabuk-do 54875 (KR); DOF Co., Ltd., Pyeongtaek-si Gyeonggi-do 17798 (KR)
(72) Inventor: KIM, Beom Gi, Wanju-gun Jeollabuk-do 55365 (KR); PARK, Sangkyu, Wanju-gun Jeollabuk-do 55365 (KR); LEE, Hyo, Wanju-gun Jeollabuk-do 55365 (KR); KIM, Myungjin, Suwon-si Gyeonggi-do 16608 (KR); HA, Jihee, Wanju-gun Jeollabuk-do 55365 (KR); AHN, Chulhyun, Hwaseong-si Gyeonggi-do 18414 (KR); JANG, Donggil, Hwaseong-si Gyeonggi-do 18441 (KR); SONG, Jaeeun, Wanju-gun Jeollabuk-do 55365 (KR); LEE, Saet Buyl, Wanju-gun Jeollabuk-do 55365 (KR); LEE, Jong Yeol, Wanju-gun Jeollabuk-do 55365 (KR)
(74) Representative: Algemeen Octrooi- en Merkenbureau B.V.
(86) International application number: PCT/KR2022/017455
(87) International publication number: WO 2023/243780

(57) **Abstract**

The present invention relates to a method for increasing the flavonoid content in a plant, comprising treating the plant with a sulfur compound; a method for producing a plant with increased flavonoid content; a plant with increased flavonoid content, produced by the production method; an antioxidant composition or food composition comprising the plant, an extract thereof or a fraction thereof; and a composition for increasing the flavonoid content in a plant, comprising a compound represented by Formula (1) or (2) as an active ingredient. The sulfur compounds S17 and 3-(2-S) of the present invention activate the flavonoid pathway, which is a repository of various functional secondary metabolites, and thus, may strongly trigger the accumulation of functional flavonoids when applied to crops, thereby efficiently improving the added value of crops.

## Description

### [Technical Field]

The present invention relates to a method for increasing the flavonoid content in a plant through treatment with a sulfur compound, and specifically, to a method for increasing the flavonoid content in a plant, comprising treating the plant with a sulfur compound; a method for producing a plant with increased flavonoid content; a plant with increased flavonoid content, produced by the production method; an antioxidant composition or food composition comprising the plant, an extract thereof or a fraction thereof; and a composition for increasing the flavonoid content in a plant.

### [Background Art]

Plants are known to produce tens of thousands of types of phytochemicals with various functional properties (anti-cancer, anti-inflammatory, and antioxidant ingredients). Among these phytochemicals, flavonoids are known to have various functional properties in addition to their excellent antioxidant properties. In plants, flavonoid substances are known to accumulate in leaves, seeds, fruits and flowers of plants to play a role in protecting plants from UV light and to be also involved in the plant's defense mechanisms through interactions with pathogenic microorganisms or insects. In addition, flavonoid-based substances have been reported to have various functional properties such as antioxidant, anti-cancer, anti-inflammatory and anti-viral properties when ingested, so that they are attracting great attention in the pharmaceutical industry and are also applied to various food industries as health supplements, food additives, natural preservatives, and the like.

Flavonoids are divided into six large groups: anthocyanins, isoflavones, flavanones, flavanols, flavonols, and flavones. Anthocyanins are pigments contained in large quantities in flowers and fruits and have the greatest ability to remove active oxygen from the body. Isoflavones have an excellent detox effect in expelling toxic substances. Meanwhile, flavonols are functional substances that resist stress, have the effects of preventing cardiovascular disease, restoring skin damage caused by ultraviolet rays and improving memory loss due to aging, and have excellent antioxidant effects, enough to be used as antioxidants.

Kaempferol, which is a member of the flavonol family, is one of the flavonoids found in fruits and vegetables, and is known to have a strong antioxidant effect and to inhibit the formation of cancer cells.

Research is continuously being attempted to increase the content of these flavonoids in plants to use them as food and medicine, but the results are still insufficient.

Under this background, as a result of diligent efforts to produce plants with increased flavonoid content, the present inventors have completed the present invention by producing Chinese cabbage, *Arabidopsis thaliana* and rice with increased flavonoid content through treatment with the low-molecular-weight sulfur compounds S17 and 3-(2-S).

### [Disclosure]

### [Technical Problem]

The present invention aims to solve the above-described problems and other problems associated therewith.

It is an exemplary object of the present invention to provide a method for increasing the flavonoid content in a plant, comprising treating the plant with a sulfur compound.

It is another exemplary object of the present invention to provide a method for producing a plant with increased flavonoid content, comprising treating the plant with a sulfur compound.

It is another exemplary object of the present invention to provide a plant with increased flavonoid content, produced by the production method.

It is another exemplary object of the present invention to provide an antioxidant composition comprising the plant, an extract thereof or a fraction thereof.

It is another exemplary object of the present invention to provide a food composition comprising the plant, an extract thereof or a fraction thereof.

It is another exemplary object of the present invention to provide a composition for increasing the flavonoid content in a plant, comprising a compound represented by following Formula (1) or (2) as an active ingredient.

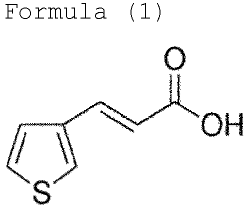

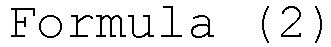

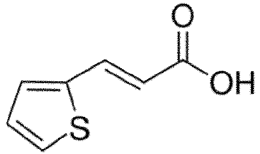

It is another exemplary object of the present invention to provide the use of the compound represented by Formula (1) or (2) for increasing the flavonoid content in a plant.

It is another exemplary object of the present invention to provide the use of the compound represented by Formula (1) or (2) for producing a plant with increased flavonoid content.

The technical problems to be achieved according to the technical idea of the invention disclosed in the present specification are not limited to the above-mentioned problems to be solved, and other problems not mentioned will be clearly understood by those skilled in the art from the description below.

### [Technical Solution]

These will be described in detail as follows. On the other hand, each description and embodiment disclosed in the present application may also be applied to each other description and embodiment. That is, all combinations of the various elements disclosed in this application fall within the scope of this application. In addition, it cannot be seen that the scope of the present application is limited by the specific descriptions described below.

As one aspect for achieving the above objects, the present invention provides a method for increasing the flavonoid content in a plant, comprising treating the plant with a sulfur compound.

The term "flavonoid" in the present invention is a type of secondary metabolite of plants or fungi, and is a substance with a 15-carbon skeleton structure consisting of two phenyl rings and a heterocyclic ring. Flavonoids are known to have antioxidant, antibacterial, anti-cancer, anti-inflammatory effects, and the like, and typically include anthocyanins, isoflavones, flavanones, flavanols, flavonols, or flavones.

In plants, flavonoids are known to accumulate in leaves, seeds, fruits and flowers of plants to play a role in protecting plants from UV light and to interact with pathogenic microorganisms or insects. In addition, flavonoid-based substances have been reported to have various functional properties such as anti-cancer, anti-inflammatory and antioxidant properties, so that they are used in various industrial fields, and are not only used in industries such as processed food additives and natural preservatives, but are also attracting attention for medical purposes due to their excellent antioxidant effect and various functional properties.

In the present invention, the flavonoid may be, but is not limited to, anthocyanin, kaempferol, cyanidin, quercetin, or isorhamnetin.

In the present invention, the "increasing the flavonoid content" includes producing flavonoids in plants that do not produce flavonoids or increasing the production content in plants that produce flavonoids.

In the present invention, the sulfur compound may be, but is not limited to, 3-(3-Thienyl)acrylic acid (hereinafter referred to as S17) represented by following Formula 1 or 3-(2-Thienyl)acrylic acid (hereinafter referred to as 3-(2-S)) represented by following Formula 2 or a derivative thereof.

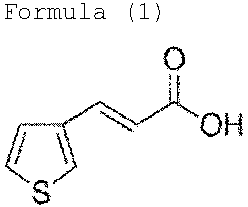

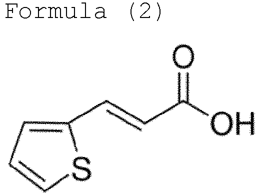

In the present invention, the concentration of the sulfur compound may be, but is not limited to, 10 µM to 250 µM, specifically 50 µM to 200 µM,50 µM to 150 µM,or 50 µM to 100 µM,more specifically 50 µM,100 µM,150 µM, or 200 µM.

In the present invention, the method for increasing the flavonoid content in a plant may further comprise treating the plant with sucrose.

The concentration of the sucrose may be 1 to 10 parts by weight, specifically 2 to 9 parts by weight, 3 to 7 parts by weight, or 4 to 6 parts by weight, more specifically 2, 3, 4, 5, 6, 7, 8, or 9 parts by weight, based on 100 parts by weight of the total medium.

In the present invention, for example, the plant may be one or more selected from Chinese cabbage, *Arabidopsis thaliana,* rice, wheat, barley, corn, soybeans, potatoes, red beans, oats, sorghum, radish, red peppers, strawberries, tomatoes, watermelons, cucumbers, cabbage, Korean melons, pumpkins, green onions, onions, and carrots.

Specifically, in the examples of the present invention, it was confirmed that when Chinese cabbage was treated with each of S17 or 3-(2-S), anthocyanins were accumulated and the kaempferol content increased, and when treated in combination with sucrose, the kaempferol content increased compared to the control group, and quercetin, isorhamnetin and cyanidin, which were not produced in the control group, were produced. In addition, it was confirmed that anthocyanins were accumulated when *Arabidopsis thaliana* was treated with both S17 or 3-(2-S) and sucrose, and anthocyanins were accumulated when rice was treated with S17 or 3-(2-S) alone.

As another aspect for achieving the above objects, the present invention provides a method for producing a plant with increased flavonoid content, comprising treating the plant with a sulfur compound.

The flavonoid, sulfur compound and plant are as described above.

In the present invention, the method for producing a plant with increased flavonoid content may further comprise treating the plant with sucrose.

The concentration of the sucrose may be 1 to 10 parts by weight, specifically 2 to 9 parts by weight, 3 to 7 parts by weight, or 4 to 6 parts by weight, more specifically 2, 3, 4, 5, 6, 7, 8, or 9 parts by weight, based on 100 parts by weight of the total medium.

As another aspect for achieving the above objects, the present invention provides a plant with increased flavonoid content, produced by the production method.

The flavonoid and plant are as described above.

As another aspect for achieving the above objects, the present invention provides an antioxidant composition comprising the plant with increased flavonoid content, an extract thereof or a fraction thereof.

The flavonoid and plant are as described above.

The term "extract" in the present invention includes the extract itself and all formulations of the extract that may be formed using the extract, such as an extract obtained by extraction treatment of plants with increased flavonoid content, a dilution or concentrate of the extract, a dried product obtained by drying the extract, a crude purified product or purified product of the extract, or mixtures thereof.

In the present invention, the extraction is not particularly limited and may be extracted according to methods commonly used in the art. Non-limiting examples of the extraction method include hot water extraction method, cold maceration extraction method, solvent extraction method, steam distillation method, elution method, compression method, ultrasonic extraction method, filtration method, reflux extraction method, and the like, which may be performed alone or using a combination of two or more methods.

The extract of the present invention is extracted from plants using an appropriate solvent, and may include, for example, a crude extract, a polar solvent-soluble extract, or a non-polar solvent-soluble extract. The type of extraction solvent used to prepare the extract is not particularly limited, and any solvent known in the art may be used. Non-limiting examples of the extraction solvent may include water; alcohols having 1 to 4 carbon atoms, such as methanol, ethanol, propanol, isopropanol, butanol, propyl alcohol and butyl alcohol; polyhydric alcohols, such as glycerin, butylene glycol and propylene glycol; hydrocarbon solvents, such as methyl acetate, ethyl acetate, acetone, benzene, hexane, diethyl ether and dichloromethane; or a mixture thereof, and may be, for example, a solvent selected from the group consisting of water, lower alcohols having 1 to 4 carbon atoms, or mixed solvents thereof.

In the present invention, the term "fraction" refers to the result obtained by performing fractionation to separate a specific component or specific group of components from a mixture containing various components.

In the present invention, the fractionation method for obtaining the fraction is not particularly limited, and may be performed according to a method commonly used in the art. It may be a solvent fractionation method performed by treatment with various solvents, an ultrafiltration fractionation method performed by passage through an ultrafiltration membrane with a certain molecular weight cut-off value, a chromatographic fractionation method, and the like, and a combination thereof. In addition, the type of solvent used to obtain the fraction in the present invention is not particularly limited, and any solvent known in the art may be used. Examples of the fractionation solvent may include hexane, dichloromethane, ethyl acetate, butanol, water, organic solvents, and the like, and mixed solvents thereof.

As another aspect for achieving the above objects, the present invention provides a food composition comprising the plant with increased flavonoid content, an extract thereof or a fraction thereof.

The flavonoid, plant, extract and fraction are as described above.

The food composition of the present invention may be prepared in various types of formulations, and unlike general drugs, it has the advantage of not having any side effects that may occur when taking the drug for a long time since it is made from food. The food composition of the present invention may be prepared in any form, and specifically, may be one or more formulations selected from the group consisting of, but is not particularly limited to, health functional food preparations such as tablets, capsules, pills, granules, liquids, powders, flakes, pastes, syrups, gels, jellies and bars, beverages, gums, and candies.

In addition, the food composition of the present invention may comprise food additives in addition to the active ingredients. Food additives may generally be understood as substances that are added to and mixed with, or infiltrated into food in order to prepare, process or preserve the food, and their safety must be guaranteed since they are consumed daily and over a long period of time with food. The food additives are classified into sweeteners, flavorants, preservatives, emulsifiers, acidulants, thickeners, and the like in terms of function, and are not particularly limited as long as they meet the purpose to be achieved by the food composition of the present invention. In addition, the food composition of the present invention may comprise, in addition to the food additives, physiological active substances or minerals known in the art for the purpose of functionality and nutritional supplementation and whose safety is guaranteed as food additives. The physiological active substances or minerals are not particularly limited as long as they meet the purpose to be achieved by the food composition of the present invention.

The food composition of the present invention may comprise the above-mentioned food additives in an effective amount to achieve the purpose of addition depending on the product type, and with respect to other food additives that may be included in the food composition of the present invention, each country's food code or food additives code may be referred to.

As another aspect for achieving the above objects, the present invention provides a composition for increasing the flavonoid content in a plant, comprising the compound represented by Formula (1) or (2) as an active ingredient.

The flavonoid and plant are as described above.

As another aspect for achieving the above objects, the present invention provides the use of the compound represented by Formula (1) or (2) for increasing the flavonoid content in a plant.

As another aspect for achieving the above objects, the present invention provides the use of the compound represented by Formula (1) or (2) for producing a plant with increased flavonoid content.

### [Advantageous Effects]

The sulfur compounds S17 and 3-(2-S) of the present invention activate the flavonoid pathway, which is a repository of various functional secondary metabolites, and thus, may strongly trigger the accumulation of functional flavonoids when applied to plants, thereby efficiently improving the added value of plants.

### [Description of Drawings]

Figure 1 shows the results of confirming anthocyanin coloration of Chinese cabbage cultivars 5923 and Whimori treated with S17 or 3-(2-S).
Figure 2 shows the analysis of flavonoid substances of Chinese cabbage cultivar Whimori (group treated with both 50 µM S17 and 4% sucrose) through HPLC.
Figure 3 shows the results of flavonoid production in Chinese cabbage cultivar 5923 according to the presence or absence of treatment with both the sulfur compound (S17 or 3-(2-S)) of the present invention and sucrose.
Figure 4 shows the results of flavonoid production in Chinese cabbage cultivar Whimori according to the presence or absence of treatment with both the sulfur compound (S17 or 3-(2-S)) of the present invention and sucrose.
Figure 5 shows the anthocyanin coloration results of *Arabidopsis thaliana* treated with both the sulfur compound (S17 or 3-(2-S)) of the present invention and sucrose.
Figure 6 shows the anthocyanin coloration results of rice cultivar purple rice treated with S17 or 3-(2-S).

### [Best Mode]

Hereinafter, the configuration and effects of the present invention will be described in more detail through examples. These examples are only for illustrating the present invention, and the scope of the present invention is not limited thereto.

### Example 1: Confirmation of increase in flavonoid content in Chinese cabbage following treatment with sulfur compound

### 1-1. Analysis of coloring effect

3-(3-Thienyl)acrylic acid (hereinafter referred to as S17) and 3-(2-Thienyl)acrylic acid (hereinafter referred to as 3-(2-S)) dissolved in DMSO at a concentration of 50 µM or 200 µM, respectively, were prepared, and seeds of commercial Chinese cabbage cultivars 5923 and Hwimori were sterilized and then germinated on 1/2 MS solid medium to prepare 4-day seedlings showing consistent growth. Afterwards, solid medium was prepared by adding 50 µM or 200 µM of S17 or 3-(2-S) to 1/2 MS solid medium containing or not containing 4 parts by weight sucrose (4%) based on 100 parts by weight of the total medium, and then, day 4 seedlings were placed on the solid medium and cultured for 10 days, and the accumulation of anthocyanins was observed with the naked eye.

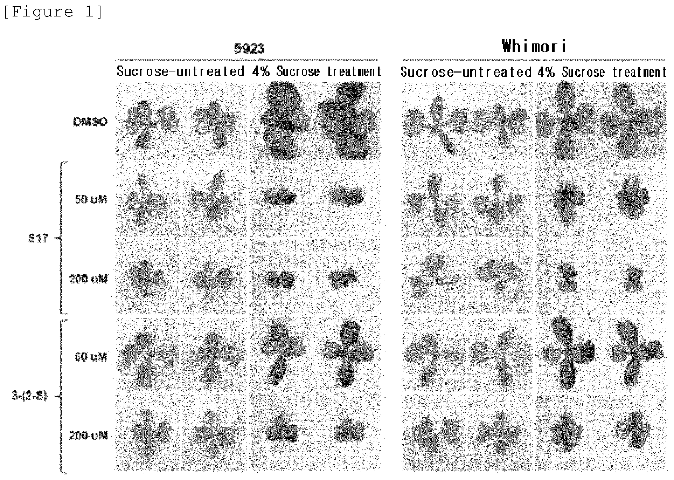

As a result, as shown in Figure 1, in the case of the cultivar 5923, it was confirmed that when treated with S17 alone, coloration appeared on the back of the leaves in the 200 µM treated group, and it was confirmed that in the group treated with both S17 and sucrose, coloration appeared on the back of the cotyledons and true leaves at 50 µM and slightly increased coloration appeared at 200 µM. Coloration was confirmed in the group treated with both 3-(2-S) at a concentration of 200 µM and 4% sucrose. It was confirmed that the cultivar Whimori showed a similar coloration pattern to the cultivar 5923 and both S17 and 3-(2-S) caused red coloration in Chinese cabbage in a concentration-dependent manner, causing anthocyanin accumulation.

### 1-2. Evaluation of flavonoid increasing effect

The leaves of the cultivars 5923 and Whimori prepared in Example 1-1 were collected and crushed, and then extracted with 50% methanol containing 2N HCl at 95°C for 2 hours, and flavonoid substances present in the form of glycosides in the plants were aglyconized and analyzed by HPLC. After the substances were separated through HPLC using a C18 column, peaks were detected at 350 nm and 520 nm, and flavonoids and anthocyanin non-glycosides in the extract were identified by comparing the retention time and UV spectrum of flavonol non-glycoside standards such as kaempferol, quercetin, myricetin and isorhamnetin, and anthocyanin non-glycoside standards such as pelargonidin, cyanidin and delphinidin, respectively, and the content of the identified flavonoid and anthocyanin substances was estimated based on the concentration of the standards and the area of the peak. As a control group, the cultivar 5923 or Whimori that was not treated with sulfur compounds was used.

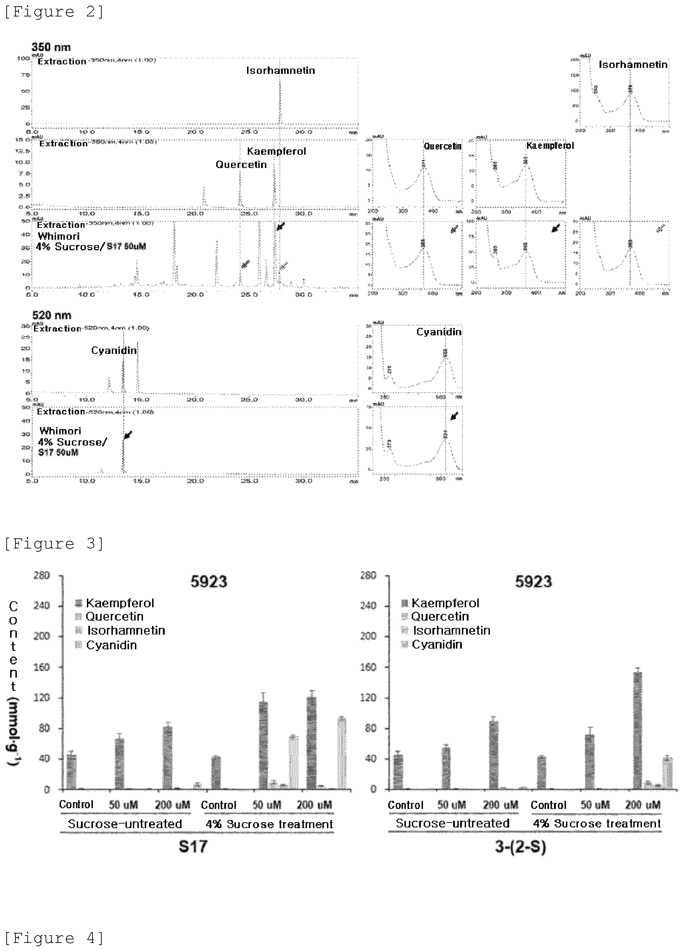

Analysis of flavonoid substances through HPLC is as shown in Figure 2 (Chinese cabbage cultirvar Hwimori, group treated with both S17 (50 µM) + 4% sucrose).

As a result, as shown in Figure 3, in the case of the cultivar 5923, it was confirmed that when treated with S17 or 3-(2-S) alone, kaempferol increased in a concentration-dependent manner, and when treated with 200 µM of S17 or 3-(2-S), kaempferol increased up to 2.5 times compared to the control group not treated with the sulfur compound, and cyanidin was accumulated.

In the case of the cultivar 5923 group treated with both S17 and 4% sucrose, the kaempferol content increased up to 6.3 times compared to the control group in the 50 µM S17 treated group, and the production of quercetin and isorhamnetin was confirmed. In the 200 µM S17 treated group, cyanidin content remarkably increased compared to the control group. In addition, it was confirmed that the anthocyanin production ability triggered in the 50 µM S17 treated group was remarkably enhanced in the 200 µM treated group. These results suggest that the accumulation of different types of flavonols was induced when treated in combination with 4% sucrose.

In the case of the cultivar 5923 group treated with both 3-(2-S) and 4% sucrose, flavonol and cyanidin accumulation patterns similar to those observed when treated with S17 were confirmed.

In the case of the cultivar Whimori, as shown in Figure 4, it was confirmed that when treated with S17 or 3-(2-S) alone, the kaempferol content remarkably increased compared to the control group, and when treated with both S17 and 4% sucrose, the kaempferol content remarkably increased in the 50 µM and 200 µM S17 treated group compared to the control group, and quercetin, isorhamnetin and cyanidin were produced. When treated with both 3-(2-S) and 4% sucrose, it was confirmed that the kaempferol content increased in a concentration-dependent manner, and quercetin, isorhamnetin and cyanidin were produced in the 200 µM 3-(2-S) treated group.

Through these results, it was confirmed that when Chinese cabbage was treated with S17 or 3-(2-S), the activation of the flavonoid metabolic pathway may be promoted, leading to the production and accumulation of flavonols and anthocyanins, and this effect may be maximized through combined treatment with sucrose.

### Example 2: Confirmation of increase in flavonoid content in Arabidopsis thaliana following treatment with sulfur compound

To confirm whether anthocyanin coloring effect occurs when *Arabidopsis thaliana,* which is a cruciferous plant such as Chinese cabbage, is treated with S17 or 3-(2-S), *Arabidopsis thaliana* was treated with S17 or 3-(2-S) alone, and each was treated with 4% sucrose.

Specifically, *Arabidopsis thaliana* seeds were germinated on 1/2 MS solid medium supplemented with 1% sucrose, and then day 6 seedlings were placed on 1/2 MS medium supplemented with each of S17 (50 µM or 200 µM) + 4% sucrose, or 3-(2-S) (50 µM or 200 µM) + 4% sucrose and cultured for 5 days to observe anthocyanin coloration.

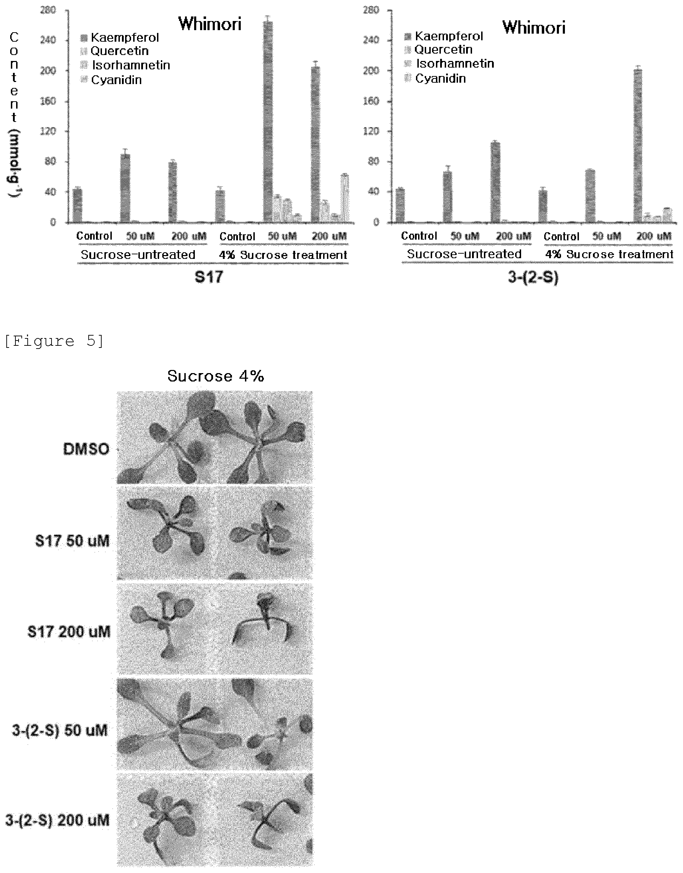

As a result, as shown in Figure 5, it was confirmed that anthocyanin coloration did not appear in the non-treated control group (DMSO), but anthocyanin coloration occurred in all treated groups and anthocyanin was produced with stronger coloring in the S17 treated group compared to the 3-(2-S) treated group.

Through these results, it was confirmed that treatment with S17 or 3-(2-S) could induce anthocyanin accumulation through combined treatment with sucrose not only in Chinese cabbage but also in other dicotyledonous plants.

### Example 3: Confirmation of increase in flavonoid content in rice following treatment with sulfur compound

To confirm whether anthocyanin coloring effects occur when not only dicotyledonous plants but also gramineous plants, which are monocotyledonous plants, are treated with S17 or 3-(2-S), seeds of the rice cultivar purple rice were sown on 1/2 MS medium containing each of S17 (40 µM) or 3-(2-S) (80 µM) and cultured for 7 days to observe anthocyanin coloration.

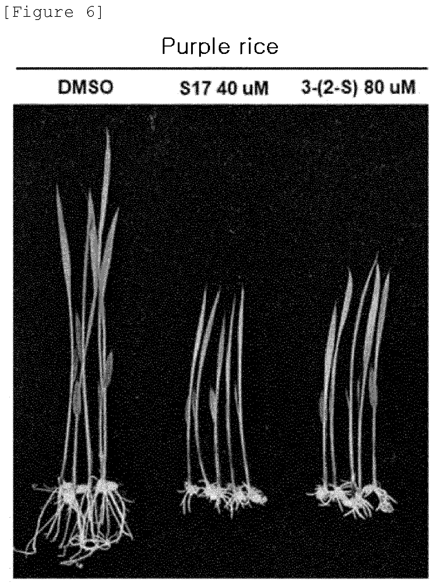

As a result, as shown in Figure **6****,** it was confirmed that coloration did not appear in the hypocotyls of the sulfur compound-untreated control group (DMSO), while anthocyanin was produced since anthocyanin coloration was strong in the hypocotyls of the S17 or 3-(2-S)-treated group. In this case, the two treated group showed similar effects at a lower concentration of S17 (40 µM) compared to the concentration of **3-(2-S)** (80 µM), suggesting that the coloration-inducing effect (anthocyanin production effect) of S17 is comparatively superior to that of 3-(2-S) even in rice.

These results indicate that anthocyanins may be produced through treatment with S17 or 3-(2-S) even in gramineous plants, which are monocotyledonous plants.

From the above description, those of ordinary skill in the technical art to which the present invention pertains will understand that the present invention may be implemented in other specific forms without changing the technical spirit or essential characteristics thereof. In this regard, it should be understood that the examples described above are illustrative and not restrictive in all respects. It should be construed that all changes or modifications derived from the meaning and scope of the claims to be described later rather than the detailed description and their equivalent concepts are included in the scope of the present invention.

## Claims

1. A method for increasing the flavonoid content in a plant, comprising treating the plant with a sulfur compound.

2. The method for increasing the flavonoid content in a plant according claim 1, wherein the sulfur compound is a compound represented by following Formula (1) or (2).

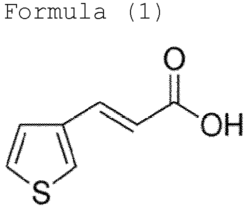

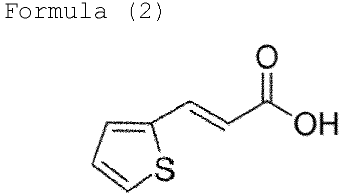

3. The method for increasing the flavonoid content in a plant according claim 1, wherein the concentration of the sulfur compound is 10 µM to 250 µM.

4. The method for increasing the flavonoid content in a plant according claim 1, further comprising treating the plant with sucrose.

5. The method for increasing the flavonoid content in a plant according claim 1, wherein the flavonoid is anthocyanin, kaempferol, cyanidin, quercetin, or isorhamnetin.

6. The method for increasing the flavonoid content in a plant according claim 1, wherein the plant is one or more selected from Chinese cabbage, *Arabidopsis thaliana,* rice, wheat, barley, corn, soybeans, potatoes, red beans, oats, sorghum, radish, red peppers, strawberries, tomatoes, watermelons, cucumbers, cabbage, Korean melons, pumpkins, green onions, onions, and carrots.

7. A method for producing a plant with increased flavonoid content, comprising treating the plant with a sulfur compound.

8. The method according claim 7, wherein the sulfur compound is a compound represented by following Formula (1) or (2).

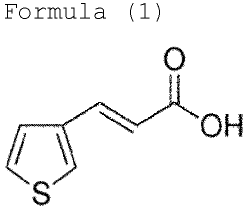

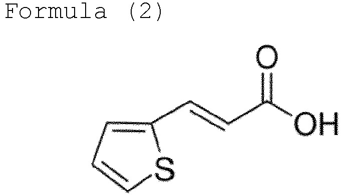

9. The method according claim 7, wherein the concentration of the sulfur compound is 10 µM to 250 µM.

10. The method according claim 7, further comprising treating the plant with sucrose.

11. The method according claim 7, wherein the flavonoid is anthocyanin, kaempferol, cyanidin, quercetin, or isorhamnetin.

12. The method according to claim 7, wherein the plant is one or more selected from Chinese cabbage, *Arabidopsis thaliana,* rice, wheat, barley, corn, soybeans, potatoes, red beans, oats, sorghum, radish, red peppers, strawberries, tomatoes, watermelons, cucumbers, cabbage, Korean melons, pumpkins, green onions, onions, and carrots.

13. A plant with increased flavonoid content, produced by the method according to any one of claims 7 to 12.

14. The plant with increased flavonoid content according claim 13, wherein the flavonoid is anthocyanin, kaempferol, cyanidin, quercetin, or isorhamnetin.

15. The plant with increased flavonoid content according claim 13, wherein the plant is one or more selected from Chinese cabbage, *Arabidopsis thaliana,* rice, wheat, barley, corn, soybeans, potatoes, red beans, oats, sorghum, radish, red peppers, strawberries, tomatoes, watermelons, cucumbers, cabbage, Korean melons, pumpkins, green onions, onions, and carrots.

16. An antioxidant composition comprising the plant with increased flavonoid content according to claim 13, an extract thereof or a fraction thereof.

17. The antioxidant composition according claim 16, wherein the flavonoid is anthocyanin, kaempferol, cyanidin, quercetin, or isorhamnetin.

18. The antioxidant composition according to claim 16, wherein the plant is one or more selected from Chinese cabbage, *Arabidopsis thaliana,* rice, wheat, barley, corn, soybeans, potatoes, red beans, oats, sorghum, radish, red peppers, strawberries, tomatoes, watermelons, cucumbers, cabbage, Korean melons, pumpkins, green onions, onions, and carrots.

19. A food composition comprising the plant with increased flavonoid content according to claim 13, an extract thereof or a fraction thereof.

20. The food composition according claim 19, wherein the flavonoid is anthocyanin, kaempferol, cyanidin, quercetin, or isorhamnetin.

21. The food composition according to claim 19, wherein the plant is one or more selected from Chinese cabbage, *Arabidopsis thaliana,* rice, wheat, barley, corn, soybeans, potatoes, red beans, oats, sorghum, radish, red peppers, strawberries, tomatoes, watermelons, cucumbers, cabbage, Korean melons, pumpkins, green onions, onions, and carrots.

22. A composition for increasing the flavonoid content in a plant, comprising a compound represented by following Formula (1) or (2) as an active ingredient.

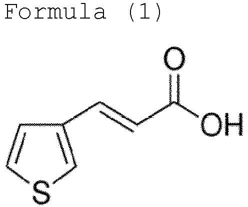

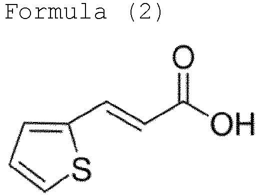

23. The composition according claim 22, wherein the flavonoid is anthocyanin, kaempferol, cyanidin, quercetin, or isorhamnetin.

24. The composition according to claim 22, wherein the plant is one or more selected from Chinese cabbage, *Arabidopsis thaliana,* rice, wheat, barley, corn, soybeans, potatoes, red beans, oats, sorghum, radish, red peppers, strawberries, tomatoes, watermelons, cucumbers, cabbage, Korean melons, pumpkins, green onions, onions, and carrots.

25. Use of a compound represented by following Formula (1) or (2) for increasing the flavonoid content in a plant.

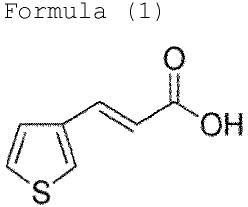

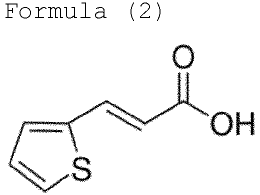

26. Use of a compound represented by following Formula (1) or (2) for producing a plant with increased flavonoid content.
